# EUROPEAN PATENT APPLICATION

(11) **EP 4 059 464 A1**
(43) Date of publication of application: **21.09.2022**
(21) Application number: 20886270.6
(22) Date of filing: 10.08.2020
(51) Int. Cl.: A61C 7/00, A61C 7/08, A61C 19/06, A61N 7/00

(54) **MOUTHPIECE-TYPE ORTHODONTIC DEVICE USING ULTRASONIC VIBRATION**

(30) Priority: 14.11.2019 KR 20190145629
(71) Applicant: Welsmeditech Co., Ltd., Chungcheongnam-do 31035 (KR)
(72) Inventor: HWANG, Kyong Won, Seoul 01197 (KR); MOON, Pyoung Su, Cheonan-si, Chungcheongnam-do 31101 (KR)
(74) Representative: Gulde & Partner
(86) International application number: PCT/KR2020/010526
(87) International publication number: WO 2021/096029

(57) **Abstract**

The present invention relates to a mouthpiece-type orthodontic device using ultrasonic vibration, in which: an ultrasonic vibrator is installed inside a mouthpiece, and ultrasonic waves emitted from the ultrasonic vibrator affect the movement of teeth requiring correction, thereby making it possible to shorten the correction time; and a vibration attenuator is attached to the outside of the mouthpiece contacting the teeth that do not need to be moved, and thus the ultrasonic vibration can be selectively transmitted only to the teeth requiring correction. The present invention comprises: a main body including a connection part and a mouthpiece inserted into the oral cavity; a first ultrasonic vibrator provided inside the mouthpiece to output ultrasonic vibrations; a control unit for controlling the operation of the first ultrasonic vibrator; and a vibration attenuator which can be mounted to the mouthpiece so as to partially attenuate the ultrasonic vibrations generated in the mouthpiece. A plurality of grooves are formed on the outside of the mouthpiece, and the vibration attenuator can be mounted thereto, thus making it possible to selectively transmit the ultrasonic vibrations only to the teeth requiring correction.

## Description

### [Technical Field]

The present invention relates to a mouthpiece type orthodontic device using ultrasonic vibrations, and more particularly, to a mouthpiece type orthodontic device using ultrasonic vibrations in which ultrasonic vibrations generated by an ultrasonic vibrator provided inside a mouthpiece have an influence on movement of a tooth to be orthodontically treated so as to reduce an orthodontic period and a vibration reduction portion is attached to an outside of the mouthpiece in contact with teeth which do not need to move so as to selectively transfer ultrasonic vibrations only to a tooth to be orthodontically treated.

### [Background Art]

Generally, orthodontics is treatment for providing normal occlusion by allowing teeth to move due to a force applied to an alveolar bone using an orthodontic device, in which osteolysis occurs at a part where an alveolar bone receives pressure and bone tissue addition occurs at a part where an alveolar bone is pulled so as to repetitively perform an alveolar bone remodeling process and perform movement of teeth.

Since movement of teeth during orthodontics is minutely performed so as not to apply an excessive force to the teeth and gum, there is a problem in which an orthodontic period is elongated according to the minute movement.

In order to solve this, a mouthpiece for orthodontics including an ultrasonic vibrator has been released. As an example of the above art, Patent Document 1 discloses a mouthpiece for orthodontics and an orthodontic device using the same in which a mouthpiece includes a wave excitation supply device such as an ultrasonic generator and the like so as to reduce a treatment period time.

Also, Patent Document 2 discloses improved dental vibration devices including a detachable matching plate for teeth remodeling and a vibration device outside an oral cavity and operationally coupled to the detachable matching plate.

Also, Patent Document 3 discloses an ultrasonic apparatus for promoting osseointegration and blood circulation of periodontal tissue for an implant, in which an ultrasonic module is mounted on a mouthpiece body and promotes curing and restoring an alveolar bone and osseointegration with an implant fixture.
Patent Document 1: Korean Patent Publication No. 10-2003-7004716 (August 9, 2003)
Patent Document 2: Korean Patent Registration No. 10-1688545 (December 15, 2016)
Patent Document 3: Korean Patent Registration No. 10-1879757 (July 19, 2018)

### [Disclosure]

### [Technical Problem]

In the arts disclosed in the above patent documents, since ultrasonic vibrations are transferred even to teeth and an alveolar bone which do not need the ultrasonic vibrations, there is a problem in which pain occurs at a part where stomatitis or periodontitis occurs.

Also, in the arts disclosed in the above patent documents, there is a problem in which only a particular part is overtreated when an alveolar osseointegration state is not uniform.

Also, in the arts disclosed in the above patent documents, there is a problem in which, in spite of a structure in which a mouthpiece is electronically controlled, it is difficult to prevent a failure occurring due to an oral secretion flowing into a device.

Also, in the arts disclosed in the above patent documents, since a mouthpiece has a fixed size, there is a problem in which it is difficult to be used by all of a variety of patients having different-sized oral cavities such as children, adults, or the like.

The present invention is directed to providing a mouthpiece type orthodontic device using ultrasonic vibrations in which a vibration reduction device is attached to a part which does not require ultrasonic vibrations so as to selectively transfer ultrasonic vibrations only to a tooth to be orthodontically treated.

The present invention is also directed to providing a mouthpiece type orthodontic device using ultrasonic vibrations configured to prevent a failure of an electronic device caused by an oral secretion of a patient.

The present invention is also directed to providing a mouthpiece type orthodontic device using ultrasonic vibrations in which a coupling module including a vibration transfer portion installed therein is detachably attached to a mouthpiece including an ultrasonic vibrator to be usable by all a variety of patients having different-sized oral cavities.

### [Technical Solution]

One aspect of the present invention provides a mouthpiece type orthodontic device using ultrasonic vibrations. The mouthpiece type orthodontic device includes a body including a mouthpiece inserted into an oral cavity and a connection portion, a first ultrasonic vibrator provided inside the mouthpiece and configured to output ultrasonic vibrations, a control portion configured to control an operation of the first ultrasonic vibrator, and a vibration reduction portion mountable on the mouthpiece to partially reduce ultrasonic vibrations generated at the mouthpiece.

The mouthpiece may be mounted on upper teeth and lower teeth of the oral cavity and include a plurality of first groove portions, and the vibration reduction portion may be detachably attached to the first groove portion.

The vibration reduction portion may include an insertion portion having a cylindrical shape to be insertable into each of the plurality of first groove portions, a head portion formed at one end of the insertion portion and having a diameter greater than that of the insertion portion, a vibration absorption member provided in the insertion portion and formed of a vibration absorption material to reduce ultrasonic vibrations, and a friction portion including frictional protrusions formed on a bottom surface of the head portion to reduce ultrasonic vibrations by applying a frictional force to the mouthpiece.

The connection portion may include a terminal formed of a silicone material and provided to be connected to the control portion, a plurality of separation prevention portions protruding from an outside of the terminal and configured to increase a coupling force of the terminal connected to the control portion, an oral secretion prevention portion provided on one side of the terminal to prevent an oral secretion from flowing to the terminal and formed to be concave toward the terminal to collect the oral secretion, and a plurality of wrinkle portions formed on the outside of the terminal to reduce ultrasonic vibrations generated at the mouthpiece.

Coupling grooves may be formed on both ends of the mouthpiece, and a plurality of coupling modules are coupled to both ends of the mouthpiece.

A second ultrasonic vibrator configured to output ultrasonic vibrations may be embedded in the coupling groove. The coupling module may include a vibration transfer portion embedded in one end thereof to protrude therefrom. The vibration transfer portion may be coupled to the coupling groove and formed of a high-density elastic material to transfer the ultrasonic vibrations output from the second ultrasonic vibrator to the coupling module.

### [Advantageous Effects]

As described above, according to a mouthpiece type orthodontic device using ultrasonic vibrations according to the present invention, since a plurality of grooves are formed in an outside of a mouthpiece so that a vibration reduction portion is mountable, an effect of selectively transferring ultrasonic vibrations only to a tooth to be orthodontically treated is obtained.

Also, according to the mouthpiece type orthodontic device using ultrasonic vibrations according to the present invention, since an oral secretion prevention portion is formed on one side of a terminal, an effect of preventing an oral secretion of a patient who wears a mouthpiece from flowing into a body or a control portion is obtained.

Also, according to the mouthpiece type orthodontic device using ultrasonic vibrations according to the present invention, since a plurality of coupling modules including a vibration transfer portion installed therein are detachably attached to a mouthpiece in which an ultrasonic vibrator is installed, an effect of being usable by a variety of patients having different-sized oral cavities such as children, adults, or the like is obtained.

### [Description of Drawings]

FIG. 1 is a perspective view according to a first embodiment of the present invention;
FIG. 2 is a side cross-sectional view according to the first embodiment of the present invention;
FIG. 3 is an exemplary view of the present invention in use according to the first embodiment of the present invention;
FIG. 4 is a side cross-sectional view in which a vibration reduction device according to the first embodiment of the present invention is partially attached;
FIG. 5 is a side cross-sectional view of the vibration reduction device according to an embodiment of the present invention;
FIG. 6 is an exemplary view of the present invention in use in which the vibration reduction device according to the first embodiment of the present invention is attached;
FIG. 7 is a perspective view according to a second embodiment of the present invention;
FIG. 8 is a side cross-sectional view in which a vibration reduction device according to the second embodiment of the present invention is partially attached; and
FIG. 9 is an exemplary view of the present invention in use in which the vibration reduction device is mounted on a coupling module according to the second embodiment of the present invention is attached.

### [Modes of the Invention]

The above and other aspects and novel features of the present invention will be further clarified by the disclosure of the specification and the attached drawings.

The term "ultrasonic wave(s)" used herein refers to a wavelength of about 2 MHz appropriate for medical use.

Also, the term "alveolar bone" used herein is thick protrusion-shaped bone tissue located at an upper jawbone and a lower jawbone and performs a function of fixing teeth and is easily remodeled so as to allow teeth to move according to orthodontic treatment.

### [First Embodiment]

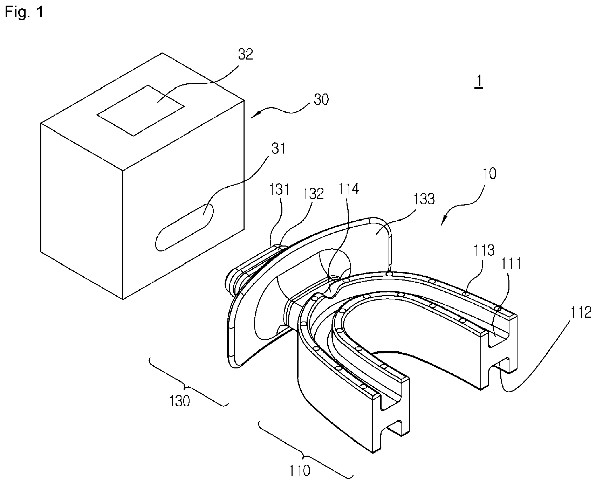

Hereinafter, a structure of a first embodiment of a mouthpiece type orthodontic device using ultrasonic vibrations according to the present invention will be described with reference to FIGS. 1 to 6.

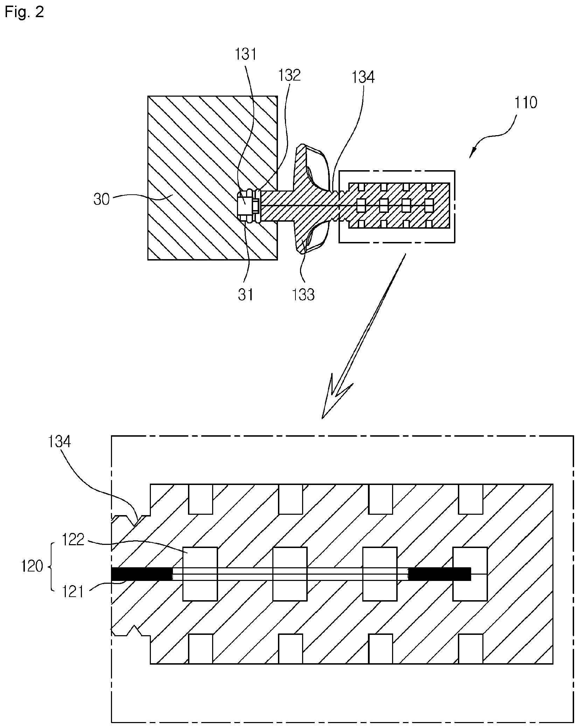

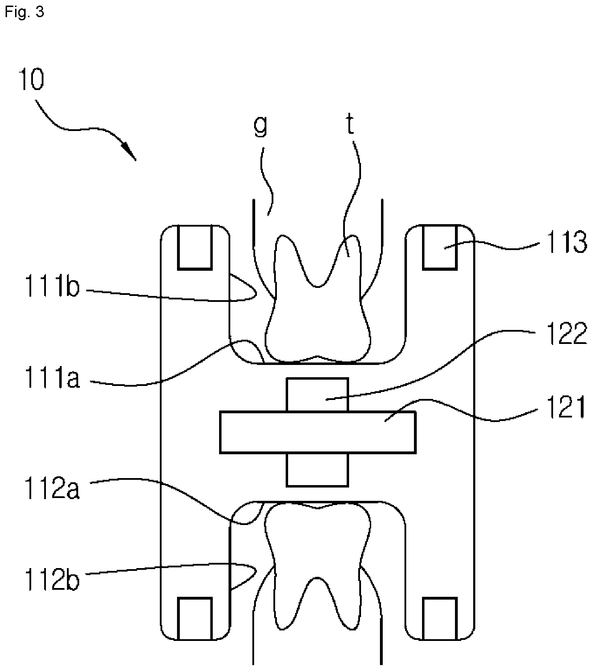

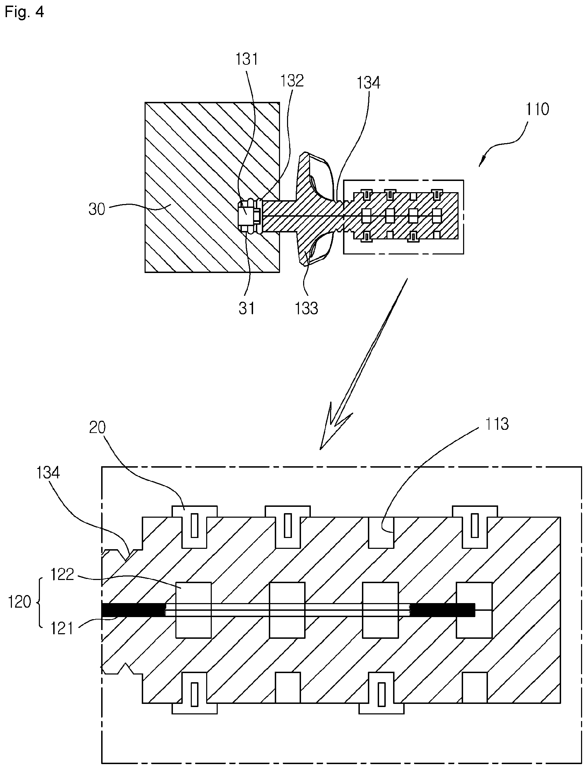

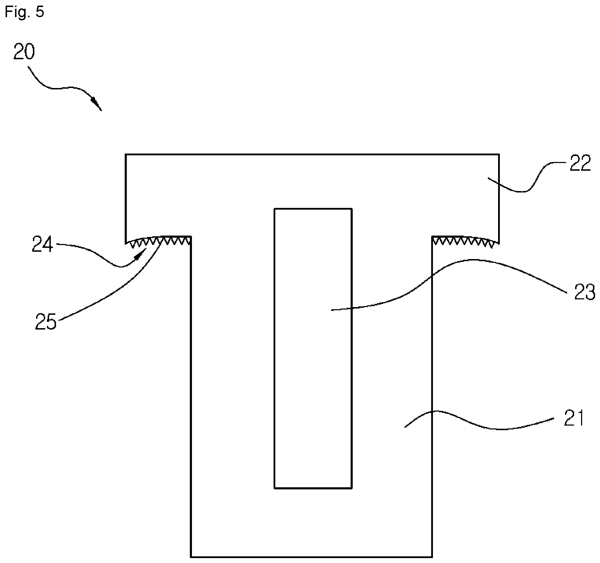

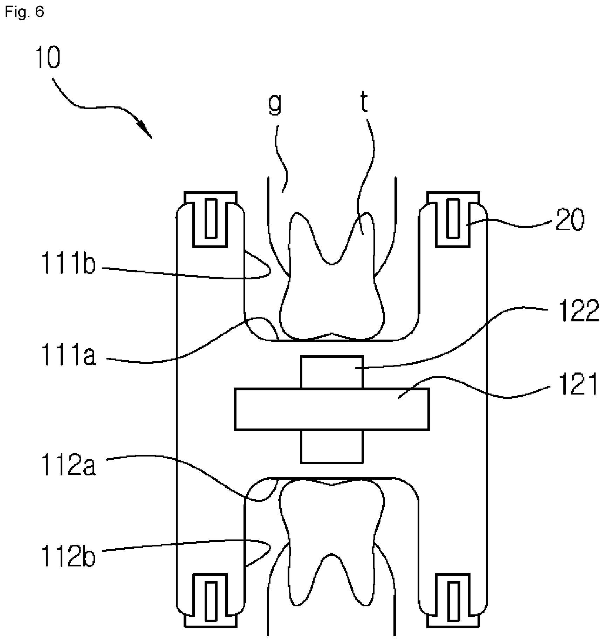

FIG. 1 is a perspective view according to a first embodiment of the present invention, FIG. 2 is a side cross-sectional view according to the first embodiment of the present invention, FIG. 3 is an exemplary view of the present invention in use according to the first embodiment of the present invention, FIG. 4 is a side cross-sectional view in which a vibration reduction device according to the first embodiment of the present invention is partially attached, FIG. 5 is a side cross-sectional view of the vibration reduction device according to the embodiment of the present invention, and FIG. 6 is an exemplary view of the present invention in use in which the vibration reduction device according to the first embodiment of the present invention is attached.

As shown in FIGS. 1 to 4, a mouthpiece type orthodontic device 1 using ultrasonic vibrations according to the present invention may include a body 10, a vibration reduction portion 20 couplable to an outside of the body 10, and a control portion 30 coupled to one side of the body 10.

The body 10 includes a mouthpiece 110 inserted into an oral cavity, a substrate portion 120 configured to generate ultrasonic vibrations inside the mouthpiece 110, and a connection portion 130 configured to electrically connect the substrate portion 120 to an outside.

The body 10 may be formed of a silicone material capable of easily transferring ultrasonic vibrations generated thereinside at about 2 MHz to teeth and an alveolar bone and be used in a medical device while the substrate portion 120 is mounted therein. Also, an exterior of the body 10 may be formed through insert injection. A method of manufacturing the body 10 is not limited thereto and any manufacturing processes in which the substrate portion 120 is insertable into and installable on an inside of the body 10 may be applied.

As shown in FIGS. 1 to 3, the mouthpiece 110 has a U-shape corresponding to teeth t and gum g of a patient and includes a first upper portion 111 in which a teeth groove is formed on an upper side to be matched with upper teeth and upper gum and a first lower portion 112 in which a teeth groove is formed on a lower side to be matched with lower teeth and lower gum.

The first upper portion 111 includes a first flat portion 111a matched with the upper teeth and a first side portion 111b facing the upper teeth and sides of the upper gum.

Also, the second lower portion 112 includes a second flat portion 112a matched with the lower teeth and a second side portion 112b facing the lower teeth and sides of the lower gum.

As shown in FIGS. 2 and 3, the substrate portion 120 is embedded in an intermediate plane between the first flat portion 111a and the second flat portion 112a and includes a substrate 121 and a first ultrasonic vibrator 122.

The substrate 121 has a U-shaped flexible material like the mouthpiece 110 and has a top surface and a bottom surface which are molded with a silicone material so as to prevent damage caused by accumulated fatigue caused by vibrations of the ultrasonic vibrator 122.

As shown in FIG. 2, the first ultrasonic vibrator 122 connected to the substrate 121 is mounted on each of the top surface and the bottom surface of the substrate 121 corresponding to positions of the teeth and the gum.

The first ultrasonic vibrator 122 is formed as an ultrasonic vibrator configured to output ultrasonic vibrations at about 2 MHz and may transfer ultrasonic vibrations to the teeth and the alveolar bone with the silicone included in the mouthpiece 110 as a medium. Accordingly, the alveolar bone may be remodeled when the teeth receive a force from an orthodontic tool and move, and an orthodontic period may be reduced as a speed of remodeling the alveolar bone is increased by the ultrasonic vibrations. Although the ultrasonic vibrator is used as the first ultrasonic vibrator 122 in the above, the present invention is not limited thereto, and any components configured to generate vibrations at about 2 MHz and that are medically usable may be applied.

As shown in FIG. 1, when worn by the patient, the mouthpiece 110 may be used without inconvenience due to an upper lip frenulum retreat groove 114 including a groove formed to allow an upper lip frenulum to be mounted therein.

As shown in FIGS. 1 to 4, a plurality of first groove portions 113 are formed in an outside of the mouthpiece 110 so that the vibration reduction portion 20 may be mounted.

As shown in FIG. 5, the vibration reduction portion 20 is approximately T-shaped, includes an insertion portion 21 inserted into each of the plurality of first groove portions 113, a head portion 22 formed to be easily held by a finger or a tool, a vibration absorption member 23 inserted into the insertion portion 21, and a friction portion 24 configured to easily reduce vibrations of the body 10, and is formed of a synthetic resin material which has an elastic modulus and strength lower than the silicone of the body 10 to effectively reduce the ultrasonic vibrations transferred from the body 10 and is medically usable.

The insertion portion 21 has an approximate cylindrical shape and is forcibly inserted into each of the plurality of first groove portions 113 not to be easily separated due to movement of the patient or the ultrasonic vibrations.

The head portion 22 has a circumferential shape having a diameter greater than that of the insertion portion 21 so as to allow the insertion portion 21 to be easily detached from or attached to the body 10.

The vibration absorption member 23 may include a polyurethane foam material for absorbing vibrations so as to effectively reduce the ultrasonic vibrations transferred to the body 10.

As shown in FIGS. 5 and 6, the friction portion 24 may be formed below the head portion 22 to become more concave in a direction from a circumferential surface of the head portion 22 toward the insertion portion 21 so as to be mounted on a surface of the mouthpiece 110 having a curvature on an outside thereof. The friction portion 24 includes micro frictional protrusions 25 formed on a surface thereof so that a frictional force is generated and effectively reduces the ultrasonic vibrations when the ultrasonic vibrations are transferred from the mouthpiece 110.

As shown in FIGS. 4 to 6, due to the above components, the vibration reduction portion 20 is selectively insertable into each of the plurality of first groove portions 113 so as to partially reduce vibrations of the mouthpiece 110. Accordingly, it is possible to selectively transfer the ultrasonic vibrations only to a tooth to be orthodontically treated.

As shown in FIGS. 1 and 2, the connection portion 130 includes a terminal 131, a separation prevention portion 132, an oral secretion prevention portion 133, and a wrinkle portion 134.

The terminal 131 is electrically connected to the substrate 121. The terminal 131 may be connected to an inside of a terminal opening 31 of the control portion 30, and vibration intensity, an output time, and the like of the first ultrasonic vibrator 122 may be adjusted by manipulating a control panel 32.

The separation prevention portion 132 doubly protruding to increase a contact force with the terminal opening 31 is formed outside the terminal 131. A fastening force may be maintained by the separation prevention portion 132 in spite of a slight tremor of the patient who receives orthodontic treatment.

Since the patient who wears the mouthpiece 110 has a difficulty in smooth swallowing of the oral secretion, when the oral secretion flows outward from the mouthpiece and into the terminal 131 or the terminal opening 31, the mouthpiece type treatment device 1 may be broken due to a short circuit. To prevent this, the oral secretion prevention portion 133 is provided on one side of the terminal 131 to rectangularly protrude and be concave toward the terminal so as to allow an oral secretion to be temporarily collected.

The wrinkle portion 134 includes a plurality of wrinkles formed on an exterior of some terminals 131 provided between the mouthpiece 110 and the oral secretion prevention portion 133 and prevents damage caused by physical fatigue generated as vibrations are transferred to the connection portion 130 and the control portion 20.

Due to the above components, the present invention obtains an effect of preventing an oral secretion of the patient who wears the mouthpiece 110 from flowing into the body 10 or the control portion 30.

### [Second Embodiment]

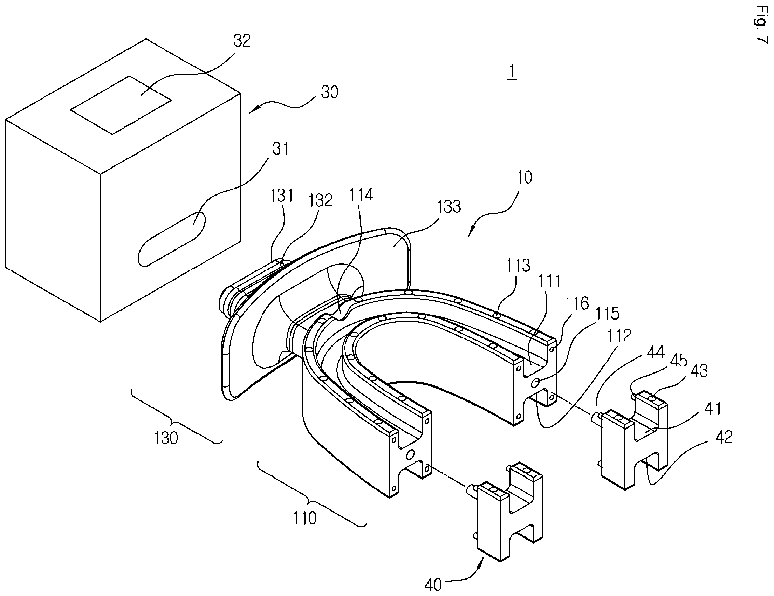

Hereinafter, a structure of a second embodiment of the mouthpiece type orthodontic device using ultrasonic vibrations according to the present invention will be described with reference to FIGS. 7 to 9. The same components as those of the first embodiment will be referred to as the same reference numerals and a repetitive description will be omitted.

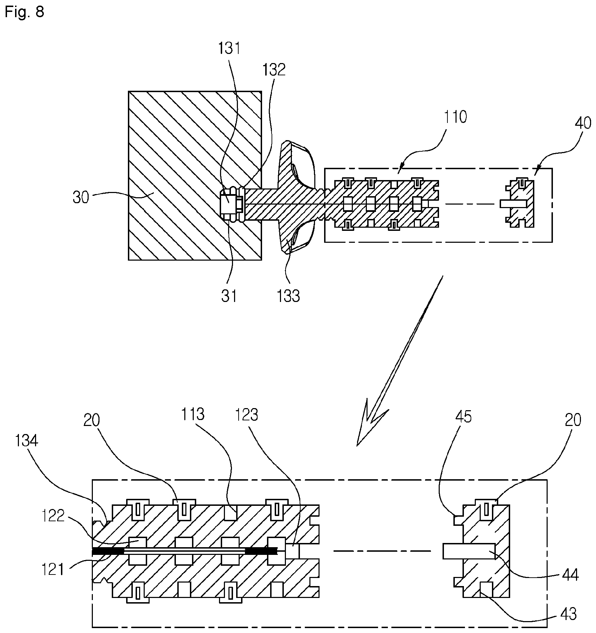

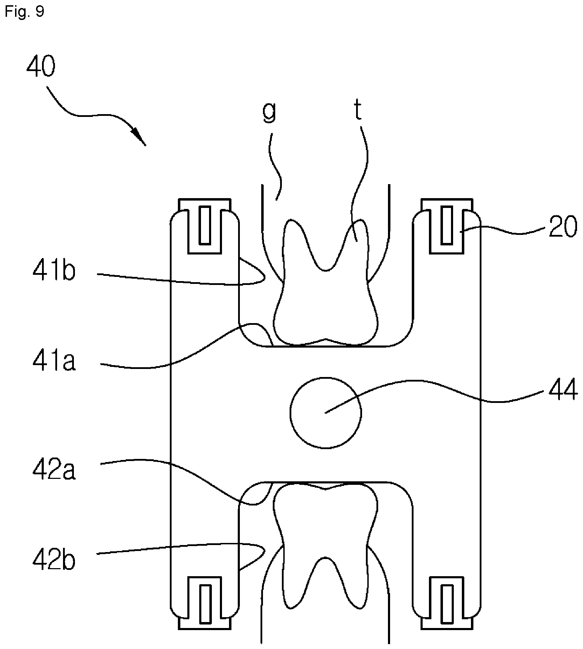

FIG. 7 is a perspective view according to a second embodiment of the present invention, FIG. 8 is a side cross-sectional view in which a vibration reduction device according to the second embodiment of the present invention is partially attached, and FIG. 9 is an exemplary view of the present invention in use in which the vibration reduction device is mounted on a coupling module according to the second embodiment of the present invention is attached.

As shown in FIGS. 7 to 9, in the mouthpiece type orthodontic device 1 using ultrasonic vibrations according to the present invention, a coupling groove 115 and an indication groove 116 are formed on both ends of the U-shaped mouthpiece 110 so as to be coupled with the coupling module 40.

The coupling groove 115 is a groove formed between the first flat portion 111a and the second flat portion 112a, and a plurality of indication grooves 116 are formed above and below the coupling groove 115.

A second ultrasonic vibrator 123 equal to the first ultrasonic vibrator 122 may be embedded in the coupling groove 115 so as to efficiently transfer ultrasonic vibrations to the coupling module 40.

A vibration transfer portion 44 is embedded in a central part of the coupling module 40 to protrude therefrom. A protruding part of the vibration transfer portion 44 may be forcibly inserted into the coupling groove 115, prevent arbitrary detachment caused by vibrations, and be formed of a synthetic resin material including high-density elastic materials so as to transfer vibrations emitted from the second ultrasonic vibrator 123 provided in the coupling groove 115 to an inside of the coupling module 40.

The coupling module 40 may be formed of the same material as the mouthpiece 110 so as to transfer the vibrations emitted from the vibration transfer portion 44 to an outside of the coupling module 40.

As shown in FIG. 7, the coupling module 40 includes a second upper portion 41 in which a teeth groove is formed on an upper side to be matched with upper teeth and upper gum and a second lower portion 42 in which a teeth groove is formed on a lower side to be matched with lower teeth and lower gum.

The second upper portion 41 includes a third flat portion 41a matched with the upper teeth and a third side portion 41b facing the upper teeth and sides of the upper gum.

Also, the second lower portion 42 includes a fourth flat portion 42a matched with the lower teeth and a fourth side portion 42b facing the lower teeth and sides of the lower gum.

Since a plurality of coupling protrusions 45 are provided on one end of the coupling module 40 and inserted into the indication groove 116, the coupling module 40 is coupled to the mouthpiece 110 without tilting. Due to the above components, teeth of a patient may be stably matched with the mouthpiece 110 and the coupling module 40.

Also, a second groove portion 43 equal to the first groove portion 113 may be formed outside the coupling module 40 to mount the vibration reduction portion 20 so as to selectively transfer ultrasonic vibrations only to a tooth to be orthodontically treated.

As shown in FIGS. 8 and 9, the vibration reduction portion 20 may be selectively inserted into each of a plurality of such second groove portions 43 so as to partially reduce the vibrations of the coupling module 40.

Due to the above components, since a plurality of such coupling modules 40 may be detached from or attached to the mouthpiece 110 and ultrasonic vibrations may be generated at the coupling module 40, the present invention may be used by a variety of patients having different-sized oral cavities.

Although the embodiment of the present invention has been described above in detail, the present invention is not limited to the above embodiment and a variety of modifications may be made without departing from the essential thereof.

### [Industrial Applicability]

The present invention is applicable to an orthodontic device configured to diagnose an array of teeth and prevent or treat malocclusion.

## Claims

1. A mouthpiece type orthodontic device using ultrasonic vibrations, comprising:
a body comprising a mouthpiece inserted into an oral cavity and a connection portion;
a first ultrasonic vibrator provided inside the mouthpiece and configured to output ultrasonic vibrations;
a control portion configured to control an operation of the first ultrasonic vibrator; and
a vibration reduction portion mountable on the mouthpiece to partially reduce ultrasonic vibrations generated at the mouthpiece.

2. The mouthpiece type orthodontic device of claim 1, wherein the mouthpiece is mounted on the upper teeth and lower teeth of the oral cavity and comprises a plurality of first groove portions,, and the vibration reduction portion is detachably attached to the first groove portion.

3. The mouthpiece type orthodontic device of claim 2, wherein the vibration reduction portion comprises:
an insertion portion having a cylindrical shape to be insertable into each of the plurality of first groove portions;
a head portion formed at one end of the insertion portion and having a diameter greater than that of the insertion portion;
a vibration absorption member provided in the insertion portion and formed of a vibration absorption material to reduce ultrasonic vibrations; and
a friction portion comprising frictional protrusions formed on a bottom surface of the head portion to reduce ultrasonic vibrations by applying a frictional force to the mouthpiece.

4. The mouthpiece type orthodontic device of claim 1, wherein the connection portion comprises:
a terminal formed of a silicone material and provided to be connected to the control portion;
a plurality of separation prevention portions protruding from an outside of the terminal and configured to increase a coupling force of the terminal connected to the control portion;
an oral secretion prevention portion provided on one side of the terminal to prevent an oral secretion from flowing to the terminal and formed to be concave toward the terminal to collect the oral secretion; and
a plurality of wrinkle portions formed on the outside of the terminal to reduce ultrasonic vibrations generated at the mouthpiece.

5. The mouthpiece type orthodontic device of claim 1, wherein coupling grooves are formed on both ends of the mouthpiece, and a plurality of coupling modules are coupled to both ends of the mouthpiece.

6. The mouthpiece type orthodontic device of claim 5, wherein a second ultrasonic vibrator configured to output ultrasonic vibrations is embedded in the coupling groove,
wherein the coupling module comprises a vibration transfer portion embedded in one end thereof to protrude therefrom, and
wherein the vibration transfer portion is coupled to the coupling groove and formed of a high-density elastic material to transfer the ultrasonic vibrations output from the second ultrasonic vibrator to the coupling module.
